# EUROPÄISCHE PATENTANMELDUNG

(11) **EP 1 120 089 A2**
(43) Veröffentlichungstag der Anmeldung: **01.08.2001**
(21) Anmeldenummer: 01250027.8
(22) Anmeldetag: 25.01.2001
(51) Int. Cl.: A61B 17/17

(54) **Führungsvorrichtung für ein chirurgisches Fräswerkzeug**

(30) Priorität: 25.01.2000 DE 10003967
(71) Anmelder: Biomet Merck Deutschland GmbH, 14167 Berlin (DE)
(72) Erfinder: Calisse, Jorge, Dr., 10785 Berlin (DE); Klas, Norbert, Dr., 64625 Bensheim (FR)
(74) Vertreter: Gross, Felix, Dr.

(57) **Zusammenfassung**

Die Erfindung betrifft eine Führungsvorrichtung (1, 10, 20) für ein einen Fräskopf nebst Antriebswelle aufweisendes Fräswerkzeug eines chirurgischen Instruments zum mechanischen Bearbeiten von Knochen, insbesondere zum Entfernen von Knochensubstanz benachbarter Wirbelknochen, gekennzeichnet durch eine Anschlageinrichtung (2.1, 5, 15, 25), welche die Bewegung des Fräswerkzeugs bei der Bearbeitung der Knochen in axialer und lateraler Richtung begrenzt.

## Beschreibung

Die Erfindung betrifft eine Führungsvorrichtung für ein einen Fräskopf nebst Antrieb aufweisenden Fräswerkzeug eines chirurgischen Instruments zum mechanischen Bearbeiten von Wirbelknochen, insbesondere zum Entfernen von Knochensubstanz zur Herstellung von Ausnehrnungen in oder zwischen benachbart angeordneten Wirbelknochen im Bereich der menschlichen Halswirbelsäule.

Es ist bekannt, das Fräswerkzeug bei derartigen chirurgischen Eingriffen einfach von Hand zu führen. Wenn ein derartiges, einen Fräskopf nebst Antriebswelle aufweisendes, Fräswerkzeug in Freihandtechnik geführt wird, besteht die große Gefahr, daß es bei der Bearbeitung der entsprechenden Wirbelknochen zu einer irreversiblen Verletzung des Rückenmarks kommen kann.

Aus der deutschen Patentanmeldung DE 39 25 488 A1 ist darüberhinaus eine Fräserausrichtvorrichtung bekannt, welche zum Führen von chirurgischen Fräsern bei der vollständigen Erneuerung eines Hüftersatzes eingesetzt wird.

Die bekannte Fräserausrichtvorrichtung umfaßt eine daran befestigte Ausrichtstange und eine die Ausrichtstange haltende Klemmeinheit, welche an den mechanisch zu bearbeitenden Knochen befestigt werden muß, um den Fräser führen zu können.

Dadurch ergibt sich in nachteiliger Weise, daß die vorgeschlagene Fräserausrichtvorrichtung grundsätzlich nur für eine Bearbeitung von länglichen und gleichzeitig auch in ausreichendem Maße frei zugänglichen Knochen einsetzbar ist. Gleichermaßen ist es von Nachteil, daß die Fräserführung lediglich einen Fixierungspunkt aufweist und dadurch unkontrollierte Abweichungen der Fräskopfbewegung von einer gewünschten Bahn nicht oder nur mit besonderer Sachwaltung zu vermeiden sind.

Ausgehends von den Mängeln des Standes der Technik liegt der Erfindung liegt deshalb die Aufgabe zugrunde, eine Führungsvorrichtung für ein chirurgisches Instrument der eingangs genannten Gattung anzugeben, durch welche ohne Gefahr einer mechanischen Verletzung des Rückenmarks durch Abtragen von Knochensubstanz ein Freiraum zwischen benachbarten Wirbelknochen herstellbar ist.

Die Aufgabe wird durch eine Führungsvorrichtung gelöst, welche die im kennzeichnenden Teil des Anspruchs 1 angegebenen Merkmale aufweist.

Die Erfindung geht von der Erkenntnis aus, daß eine erhebliche Verbesserung beim Handhaben eines Fräswerkzeugs zum Abtragen von Knochensubstanz von benachbarten Wirbelknochen ereichbar sind, wenn zwecks Vermeidung einerunerwünschten Fehlbewegung einerzum Abtragen der Knochensubstanz vorgesehenen Einrichtung eine Führungsvorrichtung eingesetzt wird, die sowohl eine axiale, d.h. in Richtung der Achse des Fräskopfes, als auch laterale Bewegung des Fräskopfes innerhalb des Raums zwischen den benachbarten Wirbelknochen, begrenzt. Dadurch wird in vorteilhafter Weise ausgeschlossen, daß der Fräskopf während des Abtragens der Knochensubstanz den inneren Bereich der Wirbelknochen erreicht und das dort befindliche Rückenmark verletzen kann.

Entsprechend der bevorzugten Ausführungsform der Erfindung ist die Führungsvorrichtung für das zur Bearbeitung benachbarter Wirbelknochen eingesetzte Fräswerkzeug als U-profilartiger Formkörper ausgebildet. Die Führungsvorrichtung weist eine Anschlageinrichtung auf, welche eine axiale und laterale Bewegung des zwischen den Profilschenkeln zu führenden Fräswerkzeugs in Richtung der mechanisch zu bearbeitenden Knochen begrenzt, wobei die Anschlageinrichtung ein Ende der Führungsvorrichtung bildet.

Erfindungsgemäß ist die Anschlageinrichtung als schmaler Steg ausgebildet und symmetrisch zur Längsachse der Führungsvorrichtung angeordnet. Für die Breite des Steges ist ein Wert vorgesehen, welcher kleiner ist als der minimalste Abstand zwischen den mechanisch zu bearbeitenden Wirbelknochen.

Die Profilschenkel verjüngen sich entsprechend der bevorzugten Ausführungsform der Erfindung in Richtung des die Anschlageinrichtung bildenden Endes des Formkörpers und sind gleichzeitig unter Beibehaltung ihrer Parallelität quer zur Richtung der Längsachse der Formkörpers eingezogen, wobei als stegförmige Anschlageinrichtung in vorteilhafter Weise die Verbindung der Profilschenkel vorgesehen ist.

Die Breite des verjüngten und eingezogenen, die laterale Bewegung des Fräskopfes begrenzenden Bereichs der Profilschenkel - und damit auch die Breite des die Axialbewegung des Fräskopfes begrenzenden Steges - weist insbesondere einen Wert auf, welcher geringer ist als der Außendurchmesser des Fräskopfes des zur Knochenbearbeitung einzusetzenden Fräswerkzeugs, damit mit für den Fräskopf des Fräswerkzeugen ein zum Knochenabtrag ausreichender Freiraum verbleibt.

Der U-profilartiger Formkörper ist entsprechend der bevorzugten Ausführungsform der Erfindung aus einem Flachmaterial, bevorzugt aus einem rostfreien Stahlblech, gebildet.

Zur Sicherung der erforderlichen mechanischen Formstabilität der Führungsvorrichtung sind erfindungsgemäß paarweise angeordnete Abstandhalter vorgesehen, welche die Endbereiche der freien Schenkel des U-Profils miteinander verbinden.

Die die Abstandhalter erstrecken sich im wesentlichen zueinander parallel und quer zur Längsachse der Führungsvorrichtung und bilden die seitliche Begrenzung einer Führungsbahn, auf welcher sich das Fräswerkzeug während der Bearbeitung der entsprechenden Wirbelknochen bewegt. Dazu ist es günstig, wenn die Abstandhalter stabförmig ausgebildet sind und der Abstand der einander gegenüberliegend angeordneten Abstandhalter im wesentlichen dem Breitenmaß des Gehäuses oder bevorzugt dem Durchmesser der Antriebswelle des zu führenden Fräswerkzeugs entspricht.

Diese konstruktive Lösung ermöglicht eine bequeme Handhabung und eine sichere Führung des Fräswerkzeugs, um bei einem Patienten zwischen Wirbelknochen einen Knochenkanal mit zueinander parallelen Seitenwandungen einzuarbeiten, welcher für weitere Behandlungen oder therapeutische Maßnahmen vorgesehen ist.

Entsprechend einer vorteilhaften Weiterbildung der Erfindung ist für die Abstandhalter jeweils eine auf ihnen drehbar angeordnete Hülse von im wesentlichen gleicher Länge vorgesehen. Dadurch wird der Reibungswiderstand zwischen den in Wirkkontakt mit der drehbar angeordneten Hülse der Abstandhalter stehenden Teile des Fräswerkzeugs bei seiner Bewegen längs seiner Führungsbahn erheblich reduziert und seine Handhabung erleichtert.

Nach einer anderen vorteilhaften Ausführungsform der Erfindung ist zwischen den paarweise vorgesehenen Abstandhaltern unter Bildung einer sich im wesentlichen quer zur Längsachse der Führungsvorrichtung erstreckenden Reihe jeweils ein Gleitblock derart angeordnet, daß der Abstand zwischen Abstandhalter und benachbartem Gleitblock im wesentlichen dem Durchmesser der Antriebswelle des zu führenden Fräswerkzeugs entspricht.

Bei symmetrischer Anordnung der Abstandshalter und Gleitblöcke entstehen zwei gegenüber der Längsachse der Führungsvorrichtung geneigte Führungsbahnen, welche jeweils den gleichen Neigungswinkel aufweisen. Wird das Fräswerkzeug wechselweise auf den einzelnen Führungsbahnen bewegt, so kann auf einfache Art und Weise ein Knochenkanal erzeugt werden, dessen Seitenwandungen sich konisch zueinander erstrecken.

In einer weiteren günstigen Ausführungsform der Erfindung sind Mittel vorgesehen, um den Abstand der aus den beiden Abstandshalterpaaren und den jeweils zwischen ihnen angeordneten Gleitblöcken gebildeten Reihen zueinander zu verändern.

Dadurch läßt sich auf einfache Weise erreichen, daß sich der Neigungswinkel der Führungsbahnen verändert, wenn die relative Position der die Fräserwelle führenden Abstandhalterpaare nebst zwischenliegenden Gleitblöcken verändert wird. Als Folge der Änderung des Neigungswinkels der Führungsbahnen wird erreicht, daß sich der konisch ausgebildete Knochenkanal in Richtung des Rückenmarks erweitert oder verjüngt.

Entsprechend einer Weiterbildung der Erfindung sind dazu in den Profilschenkeln der Führungsvorrichtung reihenförmig angeordnete Bohrungen vorgesehen. Die Bohrungsreihen erstrecken sich im wesentlichen parallel zur Längsachse der Führungsvorrichtung, wobei der Abstand benachbarter Bohrungen bevorzugt den gleichen Wert aufweist. Die Verankerung der Abstandshalter und Gleitblöcke in den Bohrungen ermöglicht auf einfache Weise eine stufenweise Änderung der Position von mindestens einer der jeweils aus zwei Abstandshaltern und einem Gleitblock gebildeten Reihe und damit eine Vergrößerung oder Verringerung des relativen Abstands zwischen beiden Reihen.

Konstruktiv besonders günstig ist es nach einer Variante der Erfindung, wenn die dem stegförmigen Anschlag benachbarte Reihe aus Abstandshaltern und Gleitblock in ihrer Position verändert wird.

Entsprechend einer zusätzlichen Variante der Erfindung ist an der Außenseite der Profilschenkel jeweils ein Haltemittel vorgesehen. Die Haltemittel sind als einen Kugelkopf tragender Zapfen ausgebildet und ermöglichen auf einfache Weise eine Fixierung der Führungsvorrichtung an einem für den operativen Eingriff notwendigen Gerät.

Andere vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt.

Es zeigen:
- Figur 1 eine bevorzugte Ausführungsform der Erfindung in Ansicht von der Seite,
- Figur 2 die Ansicht des Schnittes längs der Linie A...A gemäß Figur 1,
- Figur 3 die Darstellung der Einzelheit E gemäß Figur 1,
- Figur 4 eine vorteilhafte Weiterbildung der in Figur 1 dargestellten Ausführungsform der Erfindung, sowie
- Figur 5 eine Variante der in Figur 4 gezeigten Ausführungsform der Erfindung.

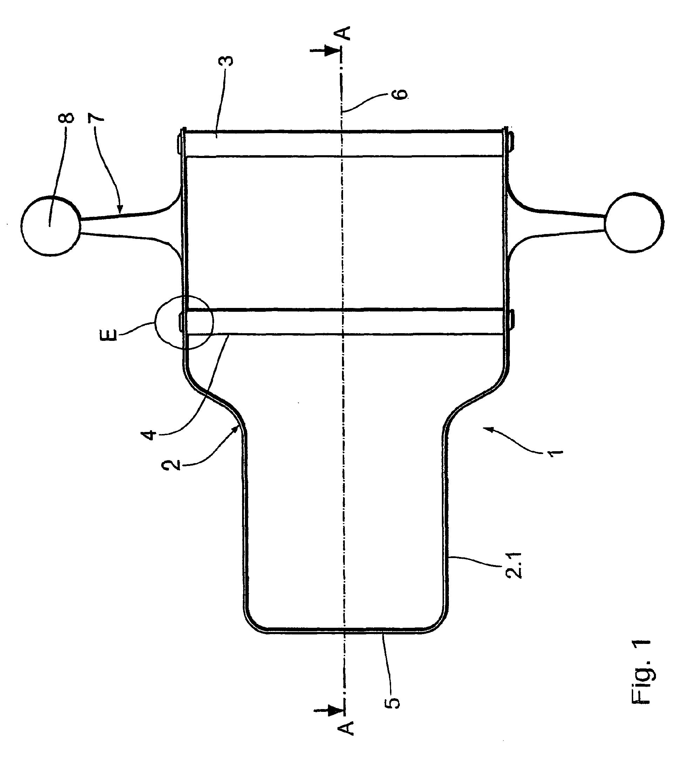

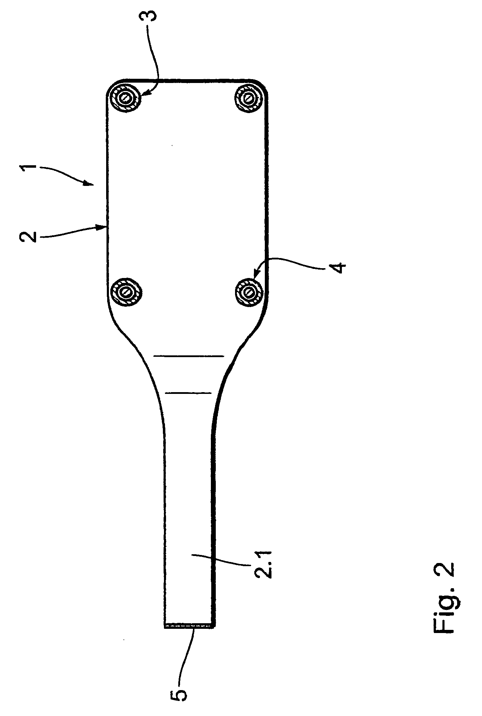

Die in den Figuren 1 und 2 gezeigte Führungsvorrichtung 1 besteht aus einem Blech aus nichtrostendem Stahl und weist ein U-förmiges Querschnittsprofil auf. Die Profilschenkel 2 sind an ihrem freien Ende durch paarweise angeordnete Abstandhalter 3, 4 verbunden. Dadurch weist die Führungvorrichtung auch bei relativ geringer Blechstärke eine ausreichende Steifigkeit auf, so daß deren Formstabilität im praktischen Einsatz nicht beeinträchtigt wird. Die Schenkel 2 des U-Profils sind am Ende des durch die Abstandshalter 3, 4 stabilisierten Bereichs eingezogen und verjüngen sich gleichzeitig. Der den eingezogenen und verjüngten Bereich 2.1 der Schenkel 2 verbindende Boden des U-Profils bildet einen Anschlag 5.

Das (nicht dargestellte) Fräswerkzeug wird innerhalb des U-Profils der Führungsvorrichtung 1 hin- und herbewegt, wobei - je nach Ausführung des Fräswerkzeug- die Antriebswelle des Fräskopfes oder das Gehäuse für den Antrieb des Fräswerkzeugs durch Wirkung der Abstandshalter 3, 4 gezwungen ist, sich auf auf einer geradlinigen Bahn längs der Achse 6 zu bewegen. Da die Bewegung des Fräskopfes durch den Anschlag 5 der Führungsvorrichtung 1 in axialer Richtung und durch die Bereiche 2.1 in lateraler Richtung begrenzt wird, ist auf vorteilhafte und zugleich einfache Art und Weise einerseits gesichert, daß der Fräskopf nicht bis in das Rückenmark vordringen kann. Andererseits kann durch die Führungswirkung der Abstandshalter 3, 4 mit dem einfach zu handhabenden Fräswerkzeug zwischen den benachbarten Wirbelknochen ein im wesentlichen quaderförmiger Freiraum sich parallel zueinander erstreckenden Seitenwänden ausgearbeitet werden.

Das Maß der Verjüngung der Profilschenkel 2 ist derart gewählt, daß die Breite des Steges 5 einen Wert aufweist, welcher kleiner ist als der minimalste Abstand zwischen den mechanisch zu bearbeitenden Wirbelknochen. Dadurch kann die Führungsvorrichtung 1 anschlagseitig zwischen die zu bearbeitenden Wirbelknochen geschoben werden und der mittig auf der Achse 6 geführte Fräskopf ragt beidseitig in genügendem Maße aus dem U-Profil heraus, um den gewünschten Freiraum zwischen den benachbarten Wirbelknochen durch Austrag von Knochenmaterial ausarbeiten zu können.

An der Außenseite der U-Profilschenkel 2 ist jeweils ein Haltezapfen 7 vorgesehen, dessen freise Ende als Kugelkopf 8 ausgebildet ist. Dieser Haltezapfen ermöglicht in vorteilhafter Weise ein Fixieren der Führungsvorrichtung 1 an bei einem operativen Eingriff eingesetzten chirurgischen Geräten, so daß die Handhabung des Fräswerkzeugs zusätzlich vereinfacht wird.

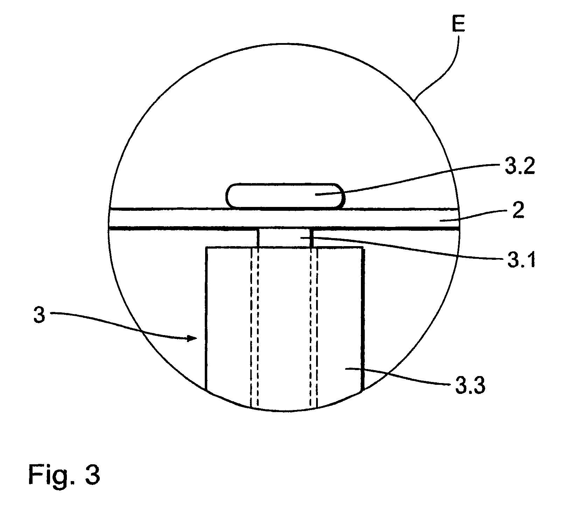

Wie in Figur 3 als Einzelheit E dargestellt, weist der Abstandhalter 3 einen Stützstab 3.1 auf, welcher beidseitig durch einen Nietkopf 3.2 in dem Profilschenkel 2 befestigt ist. Der Stützstab 3.1 bildet gleichzeitig die Achse für eine Hülse 3.3, welche um den Stützstab rotiert, wenn sich das Fräswerkzeug in dem Freiraum innerhalb des Profils der Führungsvorrichtung hin- und herbewegt. Durch die Rollbewegung der Hülse wird der Kraftaufwand für den Fräsvorgang erheblich reduziert.

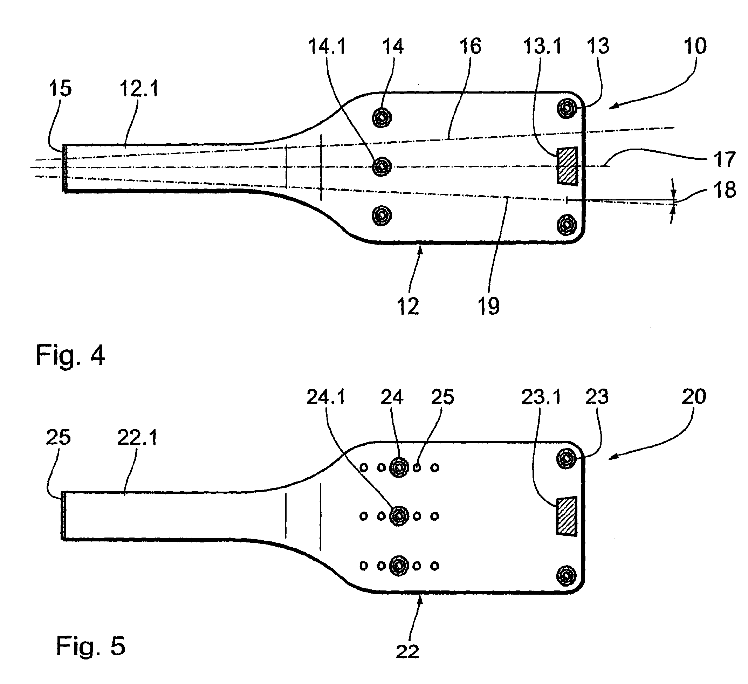

Die in Figur 4 in Schnittansicht dargestellte Führungsvorrichtung 10 weist paarweise angeordnete Abstandshalter 13, 14 auf, welche mit einem zwischen ihnen angeordneten starren bzw. drehbaren Gleitblock 13.1, 14.1 eine Reihe bilden. Je nach Wahl der Zwischenräume zwischen den Abstandhaltern 13, 14 und den Gleitböcken 13.1, 14.1 ergibt sich für das Fräswerkzeug eine Führungsbahn 16 oder 19, welche gegenüber der Längsachse 17 der Führungsvorrichtung 10 eine Neigung aufweist. Für den Neigungswinkel 18 ist ein Wert von 10° vorgesehen.

Da das Fräswerkzeug nacheinander auf zwei gegeneinander geneigter Führungsbahnen bewegt werden kann, ergibt sich auf einfache Weise die Möglichkeit, zwischen den zu bearbeitenden Wirbelknochen einen Kanal mit konisch zueinander verlaufenden Seitenwandungen auszufräsen.

Bei einem Neigungswinkel dieser Größe ist gesichert, daß die Funktion des Anschlages 15 erhalten bleibt und der Fräskopf bei seiner axialen Bewegung nicht das Rückenmark verletzen kann.

Durch Benutzung der in Figur 5 gezeigte Variante der Führungsvorrichtung 20 ist es darüberhinaus einerseits möglich, Kanäle mit konisch zueinander verlaufenden Seitenwandungen zu schaffen, welche sich in Richtung des Knocheninneren der zu bearbeitenden Wirbelknochen verjüngen oder erweitern. Andererseits kann bei begrenzter Variationsbreite ein gewünschter Konuswinkel des Kanals (vergleiche die Position 18 in Figur 4) eingestellt werden.

Dazu ist die Position der dem Anschlag 25 benachbarten, aus den Abstandshaltern 24 und dem Gleitblock 24.1 gebildeten Reihe veränderbar. Je nach Wahl der in einer sich parallel zur Längsachse der Führungsvorrichtung 20 erstreckenden Bohrungen 25 wird der Abstand zwischen den aus Abstandhaltern 23, 24 und B Gleitblöcken 23.1 und 24.1 gebildeten Reihen zur Winkeleinstellung auf einfache Weise variiert.

Die Erfindung beschränkt sich in ihrer Ausführung nicht auf die vorstehend angegebenen bevorzugten Ausführungsbeispiele. Vielmehr ist eine Anzahl von Varianten möglich, welche von der dargestellten Lösung auch bei grundsätzlich anders gearteten Ausführungen Gebrauch macht.

## Patentansprüche

1. Führungsvorrichtung (1, 10, 20) für ein einen Fräskopf nebst Antriebswelle aufweisendes Fräswerkzeug eines chirurgischen Instruments zum mechanischen Bearbeiten von Knochen, insbesondere zum Entfernen von Knochensubstanz benachbarter Wirbelknochen, gekennzeichnet durch eine Anschlageinrichtung (2.1, 5, 15, 25), welche die Bewegung des Fräswerkzeugs bei der Bearbeitung der Knochen in axialer und lateraler Richtung begrenzt.

2. Führungsvorrichtung nach Anspruch 1, dadurch gekennzeichnet, daß die Anschlageinrichtung (2.1, 5, 15, 25) einseitig die Begrenzung der Führungsvorrichtung (1, 10, 20) bildet.

3. Führungsvorrichtung nach Anspruch 2, dadurch gekennzeichnet, daß die Anschlageinrichtung einen schmalen Steg (5, 15, 25) aufweist, welcher symmetrisch zur Längsachse (6, 17) der Führungsvorrichtung (1, 10, 20) angeordnet ist.

4. Führungsvorrichtung nach Anspruch 2 oder 3, dadurch gekennzeichnet, daß die Breite des Steges (5, 15, 25) einen Wert aufweist, welcher kleiner ist als der minimalste Abstand zwischen den mechanisch zu bearbeitenden Wirbelknochen.

5. Führungsvorrichtung nach einem der vorhergehenden Ansprüche, dadurch gekennzeichnet durch eine Ausbildung als im wesentlichen U-profilartiger Formkörper (1, 10, 20), wobei die Anschlageinrichtung (5, 15, 25) die Verbindung der Profilschenkel (2, 12, 22) bildet.

6. Führungsvorrichtung nach Anspruch 5, dadurch gekennzeichent, daß die Profilschenkel (2, 12, 22) einen sich in Richtung des die Anschlageinrichtung (5, 15, 25) bildenden Endes des Formkörpers (1, 10, 20) verjüngenden Bereich (2.1) aufweisen und in diesem Bereich gleichzeitig unter Beibehaltung ihrer Parallelität quer zur Richtung der Längsachse (6, 17) des Formkörpers eingezogen sind.

7. Führungsvorrichtung nach Anspruch 6, dadurch gekennzeichnet, daß die Breite der verjüngten Profilschenkel (2.1) einen Wert aufweist, welcher geringer ist als der Außendurchmesser des Fräskopfes des Fräswerkzeugs.

8. Führungsvorrichtung nach Anspruch 6 oder 7, dadurch gekennzeichnet, daß der Formkörper (1, 10, 20) aus einem Flachmaterial gebildet ist.

9. Führungsvorrichtung nach Anspruch 6, 7 oder 8, dadurch gekennzeichnet, daß das U-Profil aus Blech, bevorzugt aus rostfreiem Stahlblech, besteht.

10. Führungsvorrichtung nach einem der Ansprüche 5 bis 9, dadurch gekennzeichnet, daß paarweise Abstandhalter (3, 4, 13, 14, 23, 24) vorgesehen sind, welche die Endbereiche der freien Schenkel (2, 12, 22) des U-Profils miteinander verbinden.

11. Führungsvorrichtung nach Anspruch 10, dadurch gekennzeichnet, daß sich die Abstandhalter (3, 4, 13, 14, 23, 24) im wesentlichen zueinander parallel und quer zur Längsachse (6, 17) der Führungsvorrichtung erstrecken.

12. Führungsvorrichtung nach einem der Ansprüche 10 oder 11, dadurch gekennzeichnet, daß die Abstandhalter (3, 4, 13, 14, 23, 24) im wesentlichen stabförmig ausgebildet sind.

13. Führungsvorrichtung nach Anspruch 12, dadurch gekennzeichnet, daß für die Abstandhalter (3, 4, 13, 14, 23, 24) jeweils eine auf ihnen drehbar angeordnete Hülse (3.3) von im wesentlichen gleicher Länge vorgesehen ist.

14. Führungsvorrichtung nach einem der Ansprüche 10 bis 13, dadurch gekennzeichnet, daß der Abstand der einander gegenüberliegend angeordneten Abstandhalter (3, 4) im wesentlichen dem Durchmesser der Antriebswelle des zu führenden Fräswerkzeugs entspricht.

15. Führungsvorrichtung nach Anspruch 10, dadurch gekennzeichnet, daß zwischen den paarweise vorgesehenen Abstandhaltern (13, 14, 23, 24) unter Bildung einer sich im wesentlichen quer zur Längsachse (17) der Führungsvorrichtung erstreckenden Reihe jeweils ein Gleitblock (13.1, 14.1, 23.1, 24.1) derart angeordnet ist, daß der Abstand zwischen Abstandhalter und benachbartem Gleitblock im wesentlichen dem Durchmesser der Antriebswelle des zu führenden Fräswerkzeugs entspricht.

16. Führungsvorrichtung nach Anspruch 15, dadurch gekennzeichnet, daß die Gleitblöcke (13.1, 14.1) und der jeweils oberhalb oder unterhalb des betreffenden Gleitblock angeordnete Abstandhalter (13, 14) eine gegenüber der Längsachse (17) der Führungsvorrichtung eine Neigung aufweisende Führungsbahn (16, 19) für das Fräswerkzeug begrenzen.

17. Führungsvorrichtung nach Anspruch 16, dadurch gekennzeichnet, daß der Neigungswinkel (18) bevorzugt einen Wert zwischen 0 und 10° aufweist.

18. Führungsvorrichtung nach Anspruch 17, dadurch gekennzeichnet, daß zwecks Variation des Neigungswinkels (18) eine der aus Abstandshaltern (24) und Gleitblock (24.1) gebildeten Reihen in Richtung der Längsachse der Führungsvorrichtung verschieblich angeordnet ist.

19. Führungsvorrichtung nach Anspruch 18, dadurch gekennzeichnet, daß in den Schenkeln (2) des U-Profils eine sich parallel zur Längsachse der Führungsvorrichtung erstreckende Reihe von Bohrungen (25) vorgesehen ist, in welchen die Abstandshalter (24) und der Gleitblock (24.1) befestigbar sind.

20. Führungsvorrichtung nach Anspruch 19, dadurch gekennzeichnet, daß der Abstand von in einer Reihe benachbart angeordneter Bohrungen (25) im wesentlichen gleich groß ist.

21. Führungsvorrichtung nach einem der Ansprüche 15 bis 20, dadurch gekennzeichnet, daß zumindest ein Gleitblock (14.1, 25.1) in seiner Befestigung drehbar ausgebildet ist.

22. Führungsvorrichtung nach einem der vorhergehenden Ansprüche, dadurch gekennzeichnet, daß an der Außenseite der Profilschenkel (2, 12, 22) jeweils ein Haltemittel (7) vorgesehen ist.

23. Führungsvorrichtung nach Anspruch 22, dadurch gekennzeichnet, daß das Haltemittel als einen Kugelkopf (8) tragender Zapfen (7) ausgebildet ist.
